(19) Europäisches Patentamt
European Patent Office
Office européen des brevets

(11) EP 4 173 624 A1

(12) EUROPEAN PATENT APPLICATION
published in accordance with Art. 153(4) EPC

(43) Date of publication:
**03.05.2023 Bulletin 2023/18**

(21) Application number: **21832922.5**

(22) Date of filing: **30.06.2021**

(51) International Patent Classification (IPC):
***A61K 31/485*** (2006.01) ***A61P 3/00*** (2006.01)
***A61P 21/00*** (2006.01)

(52) Cooperative Patent Classification (CPC):
**A61K 31/485; A61P 3/00; A61P 21/00**

(86) International application number:
**PCT/JP2021/024759**

(87) International publication number:
**WO 2022/004788 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2020 JP 2020112486**
**11.06.2021 JP 2021097684**

(71) Applicant: **Toray Industries, Inc.**
**Tokyo 103-8666 (JP)**

(72) Inventors:
- **OMORI Yu**
  **Kamakura-shi, Kanagawa 248-8555 (JP)**
- **UCHIDA Masashi**
  **Kamakura-shi, Kanagawa 248-8555 (JP)**

(74) Representative: **Kador & Partner Part mbB**
**Corneliusstraße 15**
**80469 München (DE)**

(54) **AMELIORATING AGENT OR PROPHYLACTIC AGENT FOR MUSCLE WEAKNESS SYMPTOM IN DISEASE OR SYNDROME ASSOCIATED WITH METABOLIC DISORDER**

(57) The present invention addresses the problem of providing an ameliorating agent or a prophylactic agent for a muscle weakness symptom in a disease or a syndrome associated with a metabolic disorder. The present invention provides an ameliorating agent or a prophylactic agent for a muscle weakness symptom in a disease or a syndrome associated with a metabolic disorder, the agent containing a compound having a morphinan skeleton typified by the below-mentioned compound or a pharmacologically acceptable acid addition salt thereof as an active ingredient.

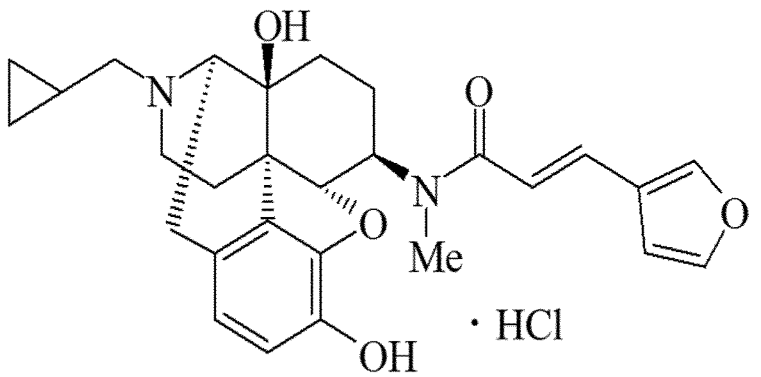

EP 4 173 624 A1

Fig. 2

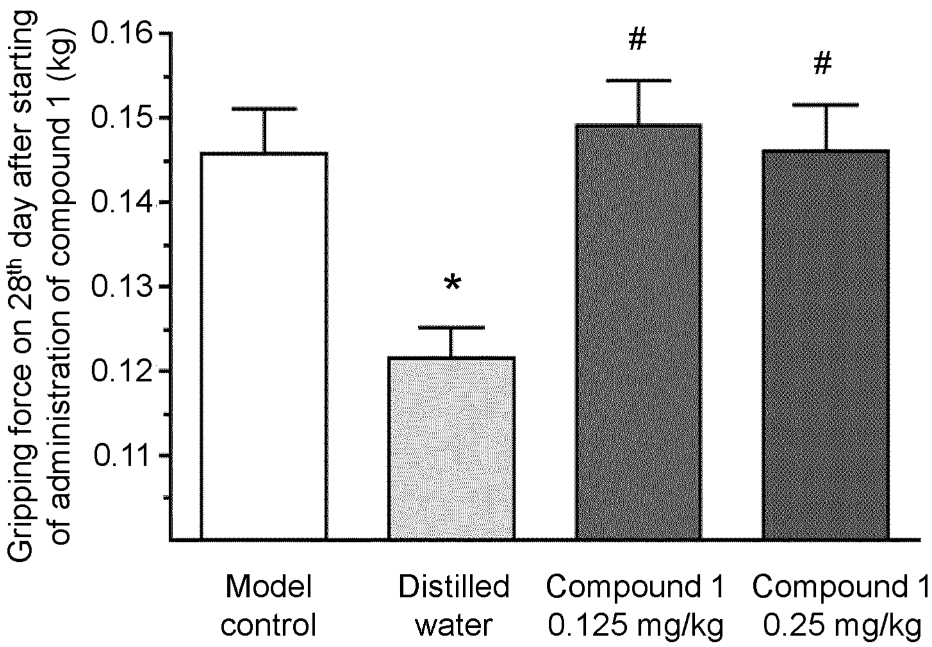
Gripping force on 28th day after starting of administration of compound 1 (kg)
0.16
0.15
0.14
0.13
0.12
0.11
#
#
*
Model control
Distilled water
Compound 1 0.125 mg/kg
Compound 1 0.25 mg/kg

## Description

### TECHNICAL FIELD

The present invention relates to an ameliorating agent or prophylactic agent for a muscle weakness symptom in diseases or syndromes associated with a metabolic disorder.

### BACKGROUND ART

Muscle weakness causes reduced locomotive ability, balance ability, physical strength and the like, risking the shortening of healthy life expectancy and transition to a state in need of nursing care. For example, muscle weakness of a hip and thigh makes it difficult to stand up or go up and down stairs, muscle weakness around a shoulder makes it difficult to lift up a heavy object, and muscle weakness of a hand makes it difficult to control fine motor skills such as holding chopsticks and the like, thereby disturbing daily activities. Additionally, muscle weakness below a knee causes legs to dangle and hence toes likely get caught with a little bump resulting in a fall and a fracture and the like associated therewith, thereby risking transition to a state in need of nursing care.

It is known that muscle weakness symptoms are caused by, for example, myogenic muscular atrophy whose cause lies in muscle itself or neurogenic muscular atrophy whose cause lies in a nervous system responsible for moving muscles (Non Patent Literature 1). It is additionally known that muscle weakness symptoms occur in, for example, diseases or syndromes associated with a metabolic disorder even when no immediate cause is found with a muscle or a nervous system (Non Patent Literature 1).

For treating a muscle weakness symptom in diseases or syndromes associated with a metabolic disorder, for example, a therapeutic drug for a causative disease or syndrome can be used. On the other hand, for drugs ameliorating a muscle weakness symptom without treating a causative disease or syndrome, for example, a non-steroidal tricyclic compound which is a selective androgen receptor modulator (Patent Literature 1) and the like are reported to have an ameliorating action on a muscle weakness symptom, but there is no launched product out on the market. For this reason, exercise therapy and nutrition therapy are practiced at present as an amelioration method and a prophylactic method for a muscle weakness symptom in diseases or syndromes associated with a metabolic disorder (Non Patent Literature 2).

It is disclosed that a compound having a morphinan skeleton or a pharmacologically acceptable acid addition salt thereof has an opioid κ receptor agonistic properties and is applicable as an analgesic agent and a diuretic agent (Patent Literature 2). Further applications as an antipruritic agent (Patent Literature 3), a cachexia therapeutic agent (Patent Literature 4), and a hypoalbuminemia improving agent (Patent Literature 5) are also disclosed.

### CITATION LIST

### PATENT LITERATURE

Patent Literature 1: International Publication No. WO 02/066475
Patent Literature 2: International Publication No. WO 93/015081
Patent Literature 3: International Publication No. WO 98/023290
Patent Literature 4: International Publication No. WO 12/105475
Patent Literature 5: International Publication No. WO 16/152965

### NON PATENT LITERATURE

Non Patent Literature 1: Lynch et al., Pharmacology & Therapeutics, 2007, Vol. 113, pp. 461-487
Non Patent Literature 2: Cruz-Jentoft et al., Age and Ageing, 2014, Vol. 43, pp. 748-759

### SUMMARY OF INVENTION

### TECHNICAL PROBLEM

However, the androgen receptor modulator is reported only as clinical trial results of an ameliorating action on a short-term muscle weakness symptom. In exercise therapies expected to ameliorate a muscle weakness symptom,

risks such as movement disorders caused by inappropriate training are associated, and patients with chronical diseases such as a heart disease, a respiratory disease, or an orthopedic disease cannot practice or continue the exercise therapy itself. Further, an ameliorating action on a long-term muscle weakness symptom of nutrition therapies is not known at present. In other words, the existing therapies limit patients who can practice, and evidence demonstrating therapeutic effects is poor, due to which there is no satisfactory therapy available currently for ameliorating a muscle weakness symptom. Under the circumstances, development of a novel ameliorating agent or prophylactic agent for a muscle weakness symptom in diseases or syndromes associated with a metabolic disorder has been awaited.

Thus, the present invention aims to provide an ameliorating agent or a prophylactic agent for a muscle weakness symptom in diseases or syndromes associated with a metabolic disorder.

SOLUTION TO PROBLEM

The present inventors conducted extensive studies to solve the above problem, and consequently found that a compound having a morphinan skeleton or a pharmacologically acceptable acid addition salt thereof has excellent ameliorating effect or prophylactic effect on a muscle weakness symptom in diseases or syndromes associated with a metabolic disorder, whereby the present invention has been accomplished.

In other words, the present invention provides an ameliorating agent or a prophylactic agent for a muscle weakness symptom in diseases or syndromes associated with a metabolic disorder, comprising, as an active ingredient, a compound represented by the following formula (I) or a pharmacologically acceptable acid addition salt thereof:

[Formula 1]

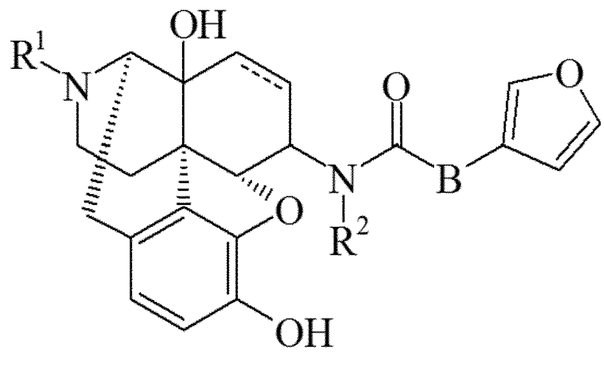

(I)

wherein a double line of a dotted line and a solid line represents a double bond or a single bond, $R^1$ represents a cycloalkylalkyl having 4 to 7 carbon atoms, $R^2$ represents a linear or branched alkyl having 1 to 5 carbon atoms, and B represents a -CH=CH-.

In the compound represented by the above formula (I), $R^1$ is preferably a cyclopropylmethyl, a cyclobutylmethyl, a cyclopentylmethyl or a cyclohexylmethyl, and $R^2$ is preferably a methyl, an ethyl or a propyl.

In the compound represented by the above formula (I), $R^1$ is more preferably a cyclopropylmethyl, $R^2$ is more preferably a methyl, and B is more preferably a *trans*-CH=CH-.

The compound represented by the above formula (I) is further preferably (-)-17-(cyclopropylmethyl)-3,14β-dihydroxy-4,5α-epoxy-6β-[N-methyl-*trans*-3-(3-furyl)acrylamide]morphinan represented by the following structural formula:

[Formula 2]

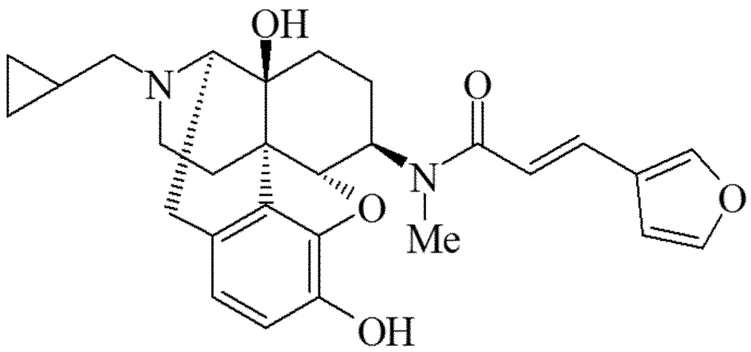

In this case, excellent ameliorating effect or prophylactic effect on a muscle weakness symptom in diseases or syndromes associated with a metabolic disorder can be expected.

Examples of the above metabolic disorder include anabolic resistance and hypercatabolism. In other words, an aspect of the present invention provides an ameliorating agent or a prophylactic agent for a muscle weakness symptom in diseases or syndromes associated with anabolic resistance, comprising, as an active ingredient, the compound represented by the above formula (I) or a pharmacologically acceptable acid addition salt thereof. Additionally, an aspect of the present invention provides an ameliorating agent or a prophylactic agent for a muscle weakness symptom in diseases or syndromes associated with hypercatabolism, comprising, as an active ingredient, the compound represented by the above formula (I) or a pharmacologically acceptable acid addition salt thereof. The above preferable aspects of the compound represented by the above formula (I) are also applied to the above aspects.

Another aspect of the present invention provides a pharmaceutical composition comprising the compound represented by the above formula (I) or a pharmacologically acceptable acid addition salt thereof and a pharmacologically acceptable carrier, for ameliorating or preventing a muscle weakness symptom in diseases or syndromes associated with a metabolic disorder. The above preferable aspects of the compound represented by the above formula (I) are also applied to the present aspect.

Further, another aspect of the present invention provides use of the compound represented by the above formula (I) or a pharmacologically acceptable acid addition salt thereof for ameliorating or preventing a muscle weakness symptom in diseases or syndromes associated with a metabolic disorder. The above preferable aspects of the compound represented by the above formula (I) are also applied to the present aspect.

Further, another aspect of the present invention provides the compound represented by the above formula (I) or a pharmacologically acceptable acid addition salt thereof for use in ameliorating or preventing a muscle weakness symptom in diseases or syndromes associated with a metabolic disorder. The above preferable aspects of the compound represented by the above formula (I) are also applied to the present aspect.

Further, another aspect of the present invention provides use of the compound represented by the above formula (I) or a pharmacologically acceptable acid addition salt thereof in the manufacture of a medicament for ameliorating or preventing a muscle weakness symptom in diseases or syndromes associated with a metabolic disorder (e.g., the ameliorating agent or the prophylactic agent for a muscle weakness symptom in diseases or syndromes associated with a metabolic disorder). The above preferable aspects of the compound represented by the above formula (I) are also applied to the present aspect.

Further, another aspect of the present invention provides a method for ameliorating or preventing a muscle weakness symptom in diseases or syndromes associated with a metabolic disorder, the method comprising a step of administering the compound represented by the above formula (I) or a pharmacologically acceptable acid addition salt thereof to a patient in need of ameliorating or preventing a muscle weakness symptom in diseases or syndromes associated with a metabolic disorder. The above preferable aspects of the compound represented by the above formula (I) are also applied to the present aspect.

## ADVANTAGEOUS EFFECTS OF INVENTION

The compound of the present invention or a pharmacologically acceptable acid addition salt thereof can ameliorate a muscle weakness symptom in diseases or syndromes associated with a metabolic disorder.

The present description encompasses the contents of descriptions and/or drawings disclosed in Japanese Patent Application Nos. 2020-112486 and 2021-097684, which are bases of the priority of the present application.

BRIEF DESCRIPTION OF DRAWINGS

[Figure 1] Figure 1 is a drawing which demonstrates impacts of compound 1 on tumor volume in malignant tumor models showing a muscle weakness symptom.

[Figure 2] Figure 2 is a drawing which demonstrates a suppressing effect of compound 1 on a muscle weakness symptom in malignant tumor models showing the muscle weakness symptom.

[Figure 3] Figure 3 is a drawing which demonstrates a suppressing effect of compound 1 on a muscle weakness symptom in senescence-accelerated models showing the muscle weakness symptom.

DESCRIPTION OF EMBODIMENTS

The ameliorating agent or the prophylactic agent for a muscle weakness symptom in diseases or syndromes associated with a metabolic disorder of the present invention comprises, as an active ingredient, the compound represented by the following formula (I) or a pharmacologically acceptable acid addition salt thereof:

[Formula 3]

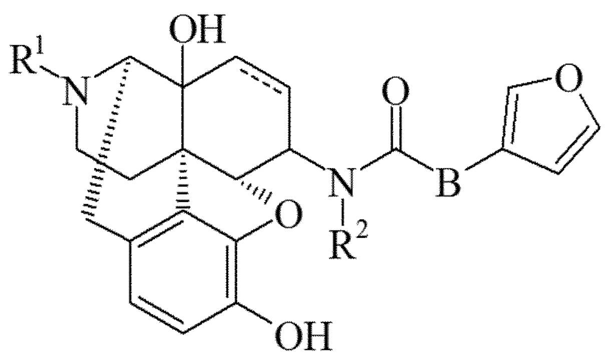

(I)

wherein a double line of a dotted line and a solid line represents a double bond or a single bond, $R^1$ represents a cycloalkylalkyl having 4 to 7 carbon atoms, $R^2$ represents a linear or branched alkyl having 1 to 5 carbon atoms, and B represents a -CH=CH-.

In the above formula (I), $R^1$ is preferably a cyclopropylmethyl, a cyclobutylmethyl, a cyclopentylmethyl or a cyclohexylmethyl, and more preferably a cyclopropylmethyl.

$R^2$ is preferably a methyl, an ethyl or a propyl, and more preferably a methyl.

B is preferably a *trans*-CH=CH-.

Further preferably, the compound represented by the formula (I) is a compound in the (-) form, wherein the double line of a dotted line and a solid line is a single bond, $R^1$ is a cyclopropylmethyl, $R^2$ is a methyl, and B is a *trans*-CH=CH-, in other words, (-)-17-(cyclopropylmethyl)-3,14β-dihydroxy-4,5α-epoxy-6β-[N-methyl-*trans*-3-(3-furyl)acrylamide] morphinan represented by the following structural formula:

[Formula 4]

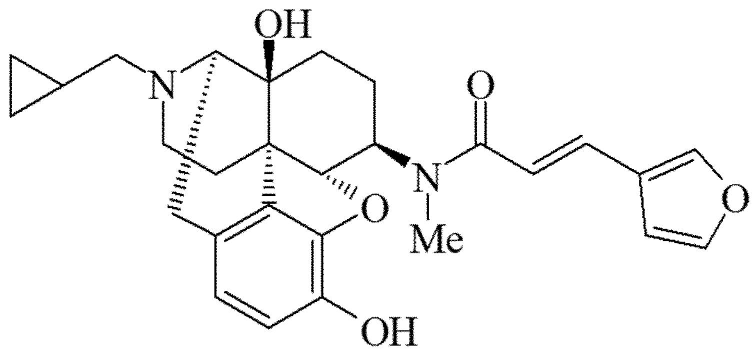

In a preferable embodiment, the compound represented by the above formula (I) or a pharmacologically acceptable acid addition salt thereof may be (-)-17-(cyclopropylmethyl)-3,14β-dihydroxy-4,5α-epoxy-6β-[N-methyl-*trans*-3-(3-furyl)acrylamide]morphinan or a pharmacologically acceptable acid addition salt thereof, and examples of an embodiment of the (-)-17-(cyclopropylmethyl)-3,14β-dihydroxy-4,5α-epoxy-6β-[N-methyl-*trans*-3-(3-furyl)acrylamide]morphinan or a pharmacologically acceptable acid addition salt thereof include (-)-17-(cyclopropylmethyl)-3,14β-dihydroxy-4,5α-epoxy-6β-[N-methyl-*trans*-3-(3-furyl)acrylamide]morphinan hydrochloride.

The following terms used in the present description are defined as follows unless otherwise stated.

Examples of the "pharmacologically acceptable acid addition salt" of the compound represented by the above formula (I) include a salt with an inorganic acid and a salt with an organic acid. Examples of the salt with an inorganic acid include hydrochloride, sulfate, nitrate, hydrobromate, hydroiodide and phosphate, and examples of the salt with an organic acid include oxalate, malonate, citrate, fumarate, lactate, malate, succinate, tartrate, acetate, trifluoroacetate, maleate, gluconate, benzoate, ascorbate, glutarate, mandelate, phthalate, methanesulfonate, ethanesulfonate, benzenesulfonate, p-toluenesulfonate, camphorsulfonate, aspartate, glutamate and cinnamate. Of these, hydrochloride, hydrobromate, phosphate, tartrate, methanesulfonate and the like are preferably used.

The compound represented by the above formula (I) or a pharmacologically acceptable acid addition salt thereof may be an anhydride or a solvate, or may form a crystal polymorphism. The solvate herein may be a hydrate or a nonhydrate, but a pharmacologically acceptable solvate is preferable.

The compound represented by the above formula (I) or a pharmacologically acceptable acid addition salt thereof can be produced, for example, in accordance with a known synthesis method (International Publication No. WO 93/015081).

The compound represented by the above formula (I) or a pharmacologically acceptable acid addition salt thereof can be used as an ameliorating agent or a prophylactic agent for a muscle weakness symptom in diseases or syndromes associated with a metabolic disorder. The above diseases or syndromes associated with a metabolic disorder are not limited to those listed below, and examples include cerebral apoplexy, chronic cardiac failure, chronic obstructive pulmonary disease, chronic kidney disease, type 2 diabetes, sepsis, osteoarthritis, osteoporosis, sarcopenia (primary sarcopenia, secondary sarcopenia), malignant tumor, cachexia, disuse syndrome, locomotive syndrome, geriatric syndrome, and acquired immune deficiency syndrome.

In the present description, the ameliorating agent for a muscle weakness symptom in diseases or syndromes associated with a metabolic disorder means a pharmaceutical product administered to a patient for the purpose of ameliorating a muscle weakness symptom in diseases associated with a metabolic disorder or syndromes associated with a metabolic disorder. The prophylactic agent for a muscle weakness symptom in diseases or syndromes associated with a metabolic disorder means a pharmaceutical product administered to a patient for the purpose of preventing onset or severity development of muscle weakness. For example, when a pharmaceutical product containing, as an active ingredient, the compound represented by the above formula (I) or a pharmacologically acceptable acid addition salt thereof is administered to a patient who develops malignant tumor, type 2 diabetes, cerebral apoplexy or the like showing a mild muscle weakness symptom or a patient with locomotive syndrome or the like showing a reduced walking speed for the purpose of preventing the progress of the muscle weakness symptom, the pharmaceutical product is encompassed in the prophylactic agent for the above muscle weakness symptoms. The muscle weakness causes reduced locomotive ability, balance ability and physical strength, thereby disabling minimal daily life activities without help such as walking, dressing and undressing clothes, and using a restroom and risking the shortening of healthy life expectancy and transition to a state in need of nursing care. These risks are expected to be reduced by the pharmacological action of the ameliorating agent or the prophylactic agent for a muscle weakness symptom in the above diseases or syndromes associated with a metabolic disorder.

In the present description, metabolic disorders mean a state where synthesis and degradation of muscle proteins are out of balance. Examples of the metabolic disorders include anabolic resistance which decreases synthesis of muscle

proteins and hypercatabolism which accelerates degradation of muscle proteins. Of the metabolic disorders, diseases or syndromes associated with anabolic resistance are not limited to those listed below, and examples include cerebral apoplexy, chronic obstructive pulmonary disease, chronic kidney disease, sarcopenia, malignant tumor, cachexia, acquired immune deficiency syndrome, chronic cardiac failure, disuse syndrome, locomotive syndrome, and geriatric syndrome. Additionally, of the metabolic disorders, diseases or syndromes associated with hypercatabolism are not limited to those listed below, and examples include cerebral apoplexy, chronic obstructive pulmonary disease, chronic kidney disease, sarcopenia, malignant tumor, cachexia, acquired immune deficiency syndrome, type 2 diabetes, sepsis, osteoarthritis, and osteoporosis. As described above, some diseases or syndromes may cause both metabolic disorders of anabolic resistance and hypercatabolism.

Anabolic resistance means a state where synthesis of muscle proteins decreases. Anabolic resistance is considered to be caused by an insufficient amount of amino acids, which are substrates of muscle proteins, associated with declined synthesis ability of muscle proteins and undernutrition, or the like. Thus, for the amelioration of a muscle weakness symptom, anabolic resistance is expected to be ameliorated by enabling an increase of maximal oxygen consumption by increasing an amount of daily movements (Chronic Respiratory Disease, 2014, Vol. 11, pp. 247-255) and suppressing muscle protein synthesis pathway inactivation signal caused by hypoxic state (The journal of physiology, 2006, Vol. 574, pp. 85-93). Additionally, for the amelioration of a muscle weakness symptom, anabolic resistance is expected to be ameliorated by enabling an increase of a food intake by increasing a basal metabolic rate (Journal of Japan Society of Nutrition and Food Science, 1993, Vol. 46, 451-458, The American Journal of Clinical Nutrition, 2013, Vol. 97, pp. 7-14) and increasing an amino acid intake from a meal.

Hypercatabolism means a state where degradation of muscle proteins accelerates. Hypercatabolism is considered to be caused by systemic inflammation, which is a biological reaction to recover from a disease and invasion. Thus, for an amelioration of a muscle weakness symptom, hypercatabolism is expected to be ameliorated by enabling an increase of a food intake by increasing an exercise load and a basal metabolic rate by increasing an amount of daily movements and suppressing the systemic inflammation reaction by improved immunocompetence (Science Advances, 2019, Vol. 5, eaau7802).

As described above, the amelioration of a muscle weakness symptom ameliorates a metabolic disorder by increasing an amount of daily movements and a basal metabolic rate, and is expected to cause a further ameliorating action on the muscle weakness symptom. Thus, the compound represented by the above formula (I) or a pharmacologically acceptable acid addition salt thereof is not limited to those listed below and more preferably used for, for example, cerebral apoplexy, sarcopenia, malignant tumor, cachexia, acquired immune deficiency syndrome, disuse syndrome, locomotive syndrome, and geriatric syndrome by which an amount of daily movements and a basal metabolic rate are reduced.

The compound represented by the above formula (I) or a pharmacologically acceptable acid addition salt thereof can ameliorate the muscle weakness symptom of skeletal muscles. The skeletal muscles herein mean muscles of head, neck, chest, abdomen and back, which are the core muscles, and muscles of upper limbs and lower limbs, which are the appendicular muscles.

The effectiveness of the compound represented by the above formula (I) or a pharmacologically acceptable acid addition salt thereof on an amelioration or prevention of a muscle weakness symptom in diseases or syndromes associated with a metabolic disorder can be evaluated based on an ameliorating effect on a muscle weakness symptom as an indicator using model animals of a disease or a syndrome. The effectiveness of the ameliorating effect on a muscle weakness symptom found by the present method on the amelioration of a muscle weakness symptom in diseases or syndromes associated with a metabolic disorder can be explained from a relation of a muscle weakness symptom and a metabolic disorder as described above. Examples of model animals of a disease or a syndrome showing the above muscle weakness symptom include a senescence-accelerated model (Lipids, 2013, Vol. 48, pp. 1135-1143.), a malignant tumor model (Oncology Reports, 2011, Vol. 25, pp. 189-193.), a type 2 diabetes model (Journal of Applied Physiology, 2019, Vol. 126, pp. 170-182), a cerebral ischemia model (Disease Models & Mechanisms, 2017, Vol. 10, pp. 787-796) and a tail suspension model of a normal animal with limited muscle use (Bioscience, 1979, Vol. 29, pp. 168-172). Further, with a patient, the effectiveness on amelioration or prevention of a muscle weakness symptom can be evaluated based on an indicator such as a gripping force and a physical function (walking distance within specified time).

With patients affected by a disease, an extremely high correlation between muscle (gripping force) weakness of the forelimbs and muscle weakness of the lower limbs and respiratory organs has been reported (Journal of Physical Therapy Science, 2011, Vol. 26, pp. 255-258, Annals of Rehabilitation Medicine, 2017, Vol. 41). Further, with disease model animals, the times at which the muscle (gripping force) of the forelimbs weakens and exercise ability decreases and the muscle of the hindlimbs weakens coincide (Journal of Cachexia, Sarcopenia and Muscle, 2018, Vol. 9, pp. 975-986, Neurotoxicology and Teratolog, 2003, Vol. 25, pp. 543-553). Thus, the gripping force can be the indicator showing the whole body muscle strength. Accordingly, using the above model animals, for example, the therapeutic effect on a muscle weakness symptom is evaluated based on the gripping force of the forelimbs measured with a grip test activity meter as an indicator, whereby an ameliorating effect on the muscle weakness symptom of the whole body

can be evaluated.

The compound represented by the above formula (I) or a pharmacologically acceptable acid addition salt thereof does not suppress, as shown in Examples to be described later, the increase of tumor volume in malignant tumor models to which A549 cells, adenocarcinomic human alveolar basal epithelial cells, have been transplanted to nude mice.

Cancer cachexia, a complication commonly found in malignant tumor patients, is one of the diseases associated with a muscle weakness symptom. Anamorelin, a drug aiming to treat cancer cachexia, increases a lean body mass in cancer cachexia patients and succeeds in ameliorating cancer cachexia symptoms but does not show an ameliorating action on a muscle weakness symptom (The Lancet Oncology, 2016, Vol. 17, pp. 519-531). Similarly, enobosarm, a drug aiming to treat cancer cachexia, also increases a lean body mass in cancer cachexia patients, but the effect thereof is not acknowledged in a test that used an amelioration of a muscle weakness symptom (amelioration of a physical function) as an indicator (The Lancet Oncology, 2013, Vol. 14, pp. 335-345). These results suggest that even therapeutic agents for body weight loss (diagnostic criterion) in cancer cachexia are not likely to be ameliorating agents for a muscle weakness symptom in cancer cachexia.

The compound represented by the above formula (I) or a pharmacologically acceptable acid addition salt thereof can be used as an ameliorating agent or a prophylactic agent for a muscle weakness symptom in diseases or syndromes associated with a metabolic disorder for mammals (e.g., human, mouse, rat, rabbit, dog, cat, cow, horse, pig and monkey).

When the compound represented by the above formula (I) or a pharmacologically acceptable acid addition salt thereof is clinically used as an ameliorating agent or a prophylactic agent for a muscle weakness symptom in diseases or syndromes associated with a metabolic disorder, the compound represented by the formula (I) or a pharmacologically acceptable salt thereof can be used as it is, or can be administered orally or parenterally by suitably mixing with, as a pharmacologically acceptable carrier, additives such as an excipient, a capsule membrane, a stabilizer, a preservative, a buffer, a solubilizer, an emulsifier, a diluent, a tonicity agent, a disintegrator, a lubricant, a coating agent, a plasticizer or a coloring agent.

Examples of the above excipient include D-mannitol, erythritol, lactose and macrogol. Examples of the above capsule membrane include gelatin and succinylated gelatin. Examples of the above stabilizer include sodium thiosulfate hydrate. Examples of the above disintegrator include crospovidone, low substituted hydroxypropylcellulose, croscarmellose sodium, carmellose calcium, and sodium carboxymethyl starch. Examples of the above lubricant include magnesium stearate, sodium stearyl fumarate and sucrose fatty acid ester. Examples of the above coating agent include hydroxypropylmethylcellulose and polyvinyl alcohol. Examples of the above plasticizer include concentrated glycerin and macrogol 400. Examples of the above coloring agent include titanium oxide, red ferric oxide, yellow ferric oxide and talc.

Further, the above ameliorating agent or prophylactic agent for a muscle weakness symptom in diseases or syndromes associated with a metabolic disorder can be produced suitably using the above carrier by a typical method. A pharmaceutical composition containing the compound represented by the above formula (I) or a pharmacologically acceptable acid addition salt thereof, and the pharmacologically acceptable carrier can also be produced in the same manner.

Examples of dosage forms when the compound represented by the above formula (I) or a pharmacologically acceptable acid addition salt thereof is orally administered as an ameliorating agent or a prophylactic agent for a muscle weakness symptom in diseases or syndromes associated with a metabolic disorder include a tablet, a capsule, an orally disintegrating agent, a powder and a granule, and examples of parenteral administration include intravenous rapid infusion, intravenous continuous infusion, intramuscular injection, subcutaneous injection, intracutaneous injection, an inhalant, an suppository, an ointment, a cream and a patch. Further, a known long-acting formulation is also acceptable.

The content of the compound represented by the formula (I) or a pharmacologically acceptable acid addition salt thereof in the above ameliorating agent or prophylactic agent for a muscle weakness symptom in diseases or syndromes associated with a metabolic disorder is not particularly limited, but can be adjusted in such a way as to contain typically 0.1 $\mu$g to 100 mg per dose. Additionally, the dosage can suitably be selected according to a patient's symptoms, age, sex, weight, administration method or the like and is typically preferably 0.1 $\mu$g to 20 mg, more preferably 1 $\mu$g to 10 mg, and further preferably 1 $\mu$g to 100 $\mu$g in terms of an amount of the compound represented by the formula (I) or a pharmacologically acceptable acid addition salt thereof per adult per day, and can be administered in one to several doses.

For complementing or enhancing the ameliorating effect or the prophylactic effect or for reducing the dosage, the above ameliorating agent or prophylactic agent for a muscle weakness symptom in diseases or syndromes associated with a metabolic disorder can further be administered in combination with one or more drugs used for treating, preventing diseases or syndromes associated with a metabolic disorder or reducing or suppressing symptoms. The drug to be combined may be a low molecular compound or a macromolecular protein, a polypeptide, an antibody or a vaccine. In this case, the ameliorating agent or the prophylactic agent can also be administered simultaneously with a drug(s) to be combined or at different times. The method for combining is to use each of the drugs in combination, or a drug combination can also be prepared. The dosage of a drug(s) to be combined can suitably be selected based on the dose

clinically used respectively. Additionally, the combination ratio of the above ameliorating agent or prophylactic agent for a muscle weakness symptom in diseases or syndromes associated with a metabolic disorder to a drug(s) to be combined can suitably be selected according to a subject to be administered, age, weight, symptoms of a subject to be administered, dosing time, dosage form, administration method and the like.

## EXAMPLES

Hereinafter, the present invention will be specifically described in detail in reference to Examples but is not limited thereto.

(Example 1) Effect of (-)-17-(cyclopropylmethyl)-3,14β-dihydroxy-4,5α-epoxy-6β-[N-methyl-*trans*-3-(3-furyl)acrylamide] morphinan hydrochloride in malignant tumor model:

On malignant tumor model animals obtained by transplanting A549 cells, adenocarcinomic human alveolar basal epithelial cells, to nude mice, an ameliorating action on a muscle weakness by administering (-)-17-(cyclopropylmethyl)-3,14β-dihydroxy-4,5α-epoxy-6β-[N-methyl-*trans*-3-(3-furyl)acrylamide]morphinan hydrochloride (hereinafter, compound 1) produced in accordance with International Publication No. WO 93/015081 was evaluated. The above model shows a muscle weakness symptom due to the malignant tumor which is one of diseases associated with a metabolic disorder. For this reason, an evaluation of an ameliorating effect and a prophylactic effect on the muscle weakness symptom is considered to be possible.

A549 cell subculture was carried out using 10% FBS-containing RPMI 1640 medium. For an evaluation of drug efficacy, 7-week old female BALB/C slc/nu/nu mice (Japan SLC, Inc.) were purchased, acclimated for 1 week and then used. The malignant tumor model animal was created as follows. In other words, $2.5 \times 10^7$ cells (suspended in FBS-free RPMI 1640 medium) of A549 cells per mouse were subcutaneously administered to the right abdomen of the mouse and transplanted. On 41$^{st}$ day from the cell transplantation, the mice were grouped in such a way that the average of tumor volumes in each group was equal. The tumor volume was determined by measuring a tumor diameter using a digital vernier caliper by the following calculating formula.

$$\text{Tumor volume (mm}^3\text{)} = \text{"major axis (mm)"} \times \text{"minor axis (mm)"} \times \text{"minor axis (mm)"} \times 1/2$$

To clarifying that the present model is a muscle weakness model, a control of a malignant tumor model animal was set. For the control of a malignant tumor model animal, an animal to which A549 cells and FBS-free RPMI 1640 medium were transplanted was used.

43$^{rd}$ to 70$^{th}$ days from cell transplantation, compound 1 was orally administered every day to the malignant tumor model animals for 28 days. The applied dose of compound 1 was 0.125 mg/kg (9 examples) or 0.25 mg/kg (9 examples). For a control of compound 1, distilled water was administered in the same manner (9 examples). Further, distilled water was administered in the same manner to the control animals for confirming that the muscle strength weakened in the malignant tumor model animals (10 examples). Using a grip test activity meter (Muromachi Kikai Co., Ltd.), a muscle strength was evaluated by measuring a gripping force of the forelimb on 28$^{th}$ day after starting administration of compound 1 (last day of the administration). The group that 0.125 mg/kg of compound 1 was administered to malignant tumor model animals was referred to as 0.125 mg/kg of compound 1 administered group, and the group that 0.25 mg/kg of compound 1 was administered to malignant tumor model animals was referred to as 0.25 mg/kg of compound 1 administered group. The group that distilled water was administered to malignant tumor model animals was referred to as distilled water administered group, and the group that A549 cells and FBS-free RPMI 1640 medium were transplanted and distilled water was administered thereto was referred to as model control group.

The results of compound 1 on the tumor volume are shown in Figure 1. The vertical axis shows the tumor volume (mean ± standard error) on 28$^{th}$ day after starting administration of compound 1. "Model control" on the horizontal axis shows model control group, "Distilled water" shows distilled water administered group, "Compound 1 0.125 mg/kg" shows 0.125 mg/kg of compound 1 administered group, and "Compound 1 0.25 mg/kg" shows 0.25 mg/kg of compound 1 administered group. * mark shows that there is a statistical significance in the comparison between the model control group and the distilled water administered group (t-test)

(*: $p<0.05$).

The results of compound 1 on the gripping force are shown in Figure 2. The vertical axis shows gripping force (mean ± standard error) on 28$^{th}$ day after starting administration of compound 1. "Model control" on the horizontal axis shows model control group, "Distilled water" shows distilled water administered group, "Compound 1 0.125 mg/kg"

shows 0.125 mg/kg of compound 1 administered group, and "Compound 1 0.25 mg/kg" shows 0.25 mg/kg of compound 1 administered group. * mark shows that there is a statistical significance in the comparison between the model control group and the distilled water administered group (t-test) (*: $p<0.05$). # mark shows that there are statistical significances in the comparison between the distilled water administered group and the different concentrations of compound 1 administered groups (Dunnett's multiple comparisons test) (#: $P<0.05$).

The results of Figure 1 showed a statistically significant increase in the tumor volume in the distilled water administered group compared with the model control group. The compound 1 administered groups in both doses did not show any suppressing action on the tumor volume increase.

The results of Figure 2 showed a statistically significant weakness in the gripping force (muscle strength) in the distilled water administered group compared with the model control group. Thus, it was revealed that the malignant tumor models obtained by transplanting A549 cells to nude mice showed the gripping force (muscle strength) weakness symptom. To this significant weakness in the gripping force (muscle strength), the compound 1 administered groups in both doses showed a statistically remarkable increasing action on the gripping force (muscle strength) compared with the distilled water administered group, and the gripping force was ameliorated to the extent that is equal to the gripping force (muscle strength) of the model control group.

(Example 2) Effect of (-)-17-(cyclopropylmethyl)-3,14β-dihydroxy-4,5α-epoxy-6β-[N-methyl-*trans*-3-(3-furyl)acrylamide] morphinan hydrochloride in senescence-accelerated model:

On senescence-accelerated models, SAMP8 mice, an ameliorating action on a muscle weakness by administering (-)-17-(cyclopropylmethyl)-3,14β-dihydroxy-4,5α-epoxy-6β-[N-methyl-*trans*-3-(3-furyl)acrylamide]morphinan hydrochloride (hereinafter, compound 1) produced in accordance with International Publication No. WO 93/015081 was evaluated. The above model shows a muscle weakness symptom due to geriatric syndrome which is one of diseases associated with a metabolic disorder. For this reason, an evaluation of an ameliorating effect and a prophylactic effect on the muscle weakness symptom is considered to be possible.

For the evaluation of drug efficacy, 7-week old male SAMP8/TaSlc mice (Japan SLC, Inc.) were purchased, then kept for 9 months and used for the test. For the total of 3 days from 3 days to 1 day before administration of the test substance, a gripping force of the forelimb was measured using a grip test activity meter, an average value of the 3 days was calculated, and then the mice were grouped in such a way that the average of muscle strengths in each group was equal. To clarifying that the present model is a muscle weakness model, a control of a senescence-accelerated model animal was set. For the control of a senescence-accelerated model animal, 7-week old male SAMPR1/TaSlc mice (Japan SLC, Inc.) were purchased, kept under the same conditions as the SAMP8 mice, and used for the test.

Compound 1 was orally administered to SAMP8 mice every day for 28 days. The applied dose of compound 1 was 0.125 mg/kg (10 examples) or 0.25 mg/kg (13 examples). For a control of compound 1, distilled water was administered in the same manner (11 examples). Further, distilled water was administered in the same manner to the control animals for confirming that the muscle strength weakened in the malignant tumor model animals (13 examples). Using a grip test activity meter, the muscle strength was evaluated by measuring a gripping force of the forelimb on $28^{th}$ day after starting administration of compound 1 (last day of the administration). The group that 0.125 mg/kg of compound 1 was administered to SAMP8 mice was 0.125 mg/kg of compound 1 administered group, and the group that 0.25 mg/kg of compound 1 was administered to SAMP8 mice was 0.25 mg/kg of compound 1 administered group. The group that distilled water was administered to SAMP8 mice was distilled water administered group, and the group that distilled water was administered to SAMR1 mice was model control group.

The results of compound 1 on the gripping force are shown in Figure 3. The vertical axis shows gripping force (mean ± standard error) on $28^{th}$ day after starting administration of compound 1. "Model control" on the horizontal axis shows model control group, "Distilled water" shows distilled water administered group, "Compound 1 0.125 mg/kg" shows 0.125 mg/kg of compound 1 administered group, and "Compound 1 0.25 mg/kg" shows 0.25 mg/kg of compound 1 administered group. * mark shows that there is a statistical significance in the comparison between the model control group and the distilled water administered group (t-test) (*: $p<0.05$). # mark shows that there are statistical significances in the comparison between the distilled water administered group and the different concentrations of compound 1 administered groups (Williams' multiple comparisons test) (#: $P<0.05$).

The results of Figure 3 showed a statistically significant weakness in the gripping force (muscle strength) in the distilled water administered group compared with the model control group. Thus, it was revealed that SAMP8 mice, the senescence-accelerated models, showed the gripping force (muscle strength) weakness symptom. To this significant weakness in the gripping force (muscle strength), the compound 1 administered groups in both doses showed a statistically remarkable increasing action on the gripping force (muscle strength) compared with the distilled water administered group.

The above results revealed that the compound represented by the formula (I) or a pharmacologically acceptable acid addition salt thereof shows the remarkable symptom ameliorating effect on the muscle weakness symptom. Further,

as the ameliorating action on the muscle weakness symptom has a potential of ameliorating a metabolic disorder, it was revealed that the compound represented by the formula (I) or a pharmacologically acceptable acid addition salt thereof has a potential of improving an ameliorating effect on a muscle weakness symptom in diseases or syndromes associated with a metabolic disorder.

INDUSTRIAL APPLICABILITY

The compound of the present invention or a pharmacologically acceptable acid addition salt thereof has an ameliorating effect and the like on a muscle weakness symptom in diseases or syndromes associated with a metabolic disorder, thereby being useful in the pharmaceutical field.

All of the publications, patents and patent applications cited herein are deemed to be incorporated by reference per se into the present description.

## Claims

1. An ameliorating agent or a prophylactic agent for a muscle weakness symptom in diseases or syndromes associated with a metabolic disorder, comprising, as an active ingredient, a compound represented by the following formula (I) or a pharmacologically acceptable acid addition salt thereof:

[Formula 1]

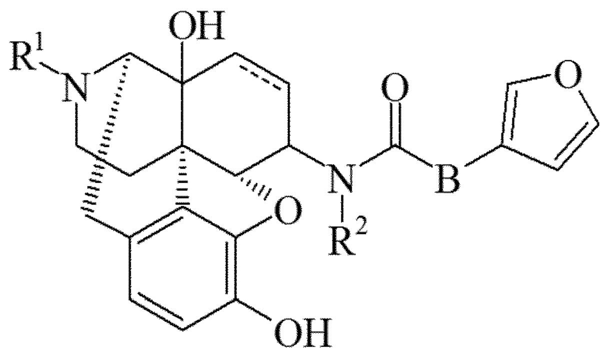

(I)

wherein a double line of a dotted line and a solid line represents a double bond or a single bond, $R^1$ represents a cycloalkylalkyl having 4 to 7 carbon atoms, $R^2$ represents a linear or branched alkyl having 1 to 5 carbon atoms, and B represents a -CH=CH-.

2. The ameliorating agent or the prophylactic agent according to claim 1, wherein $R^1$ is a cyclopropylmethyl, a cyclobutylmethyl, a cyclopentylmethyl or a cyclohexylmethyl, and $R^2$ is a methyl, an ethyl or a propyl.

3. The ameliorating agent or the prophylactic agent according to claim 1, wherein $R^1$ is a cyclopropylmethyl, $R^2$ is a methyl, and B is a *trans*-CH=CH-.

4. The ameliorating agent or the prophylactic agent according to claim 1, wherein the compound represented by the above formula (I) is (-)-17-(cyclopropylmethyl)-3,14β-dihydroxy-4,5α-epoxy-6β-[N-methyl-*trans*-3-(3-furyl)acrylamide]morphinan represented by the following structural formula:

[Formula 2]

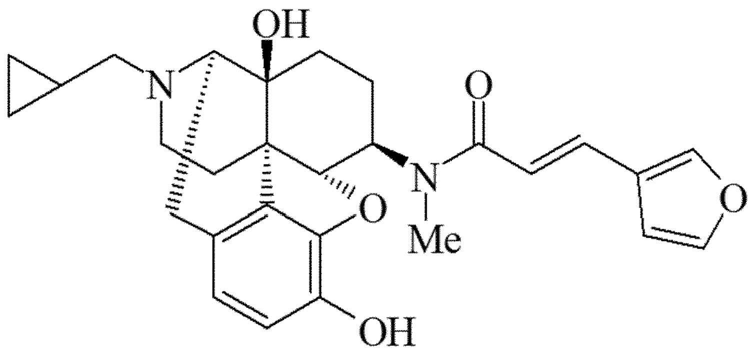

5. The ameliorating agent or the prophylactic agent according to any one of claims 1 to 4, wherein the metabolic disorder is anabolic resistance.

6. The ameliorating agent or the prophylactic agent according to any one of claims 1 to 4, wherein the metabolic disorder is hypercatabolism.

Fig. 1

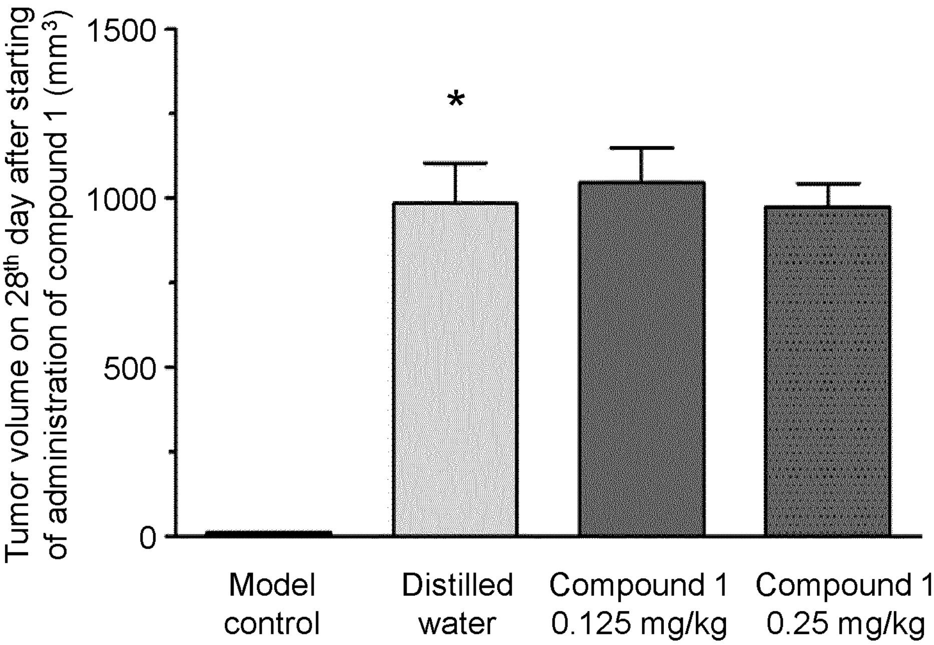

Tumor volume on 28th day after starting of administration of compound 1 (mm3)
1500
1000
500
0
*
Model control
Distilled water
Compound 1 0.125 mg/kg
Compound 1 0.25 mg/kg

Fig. 2

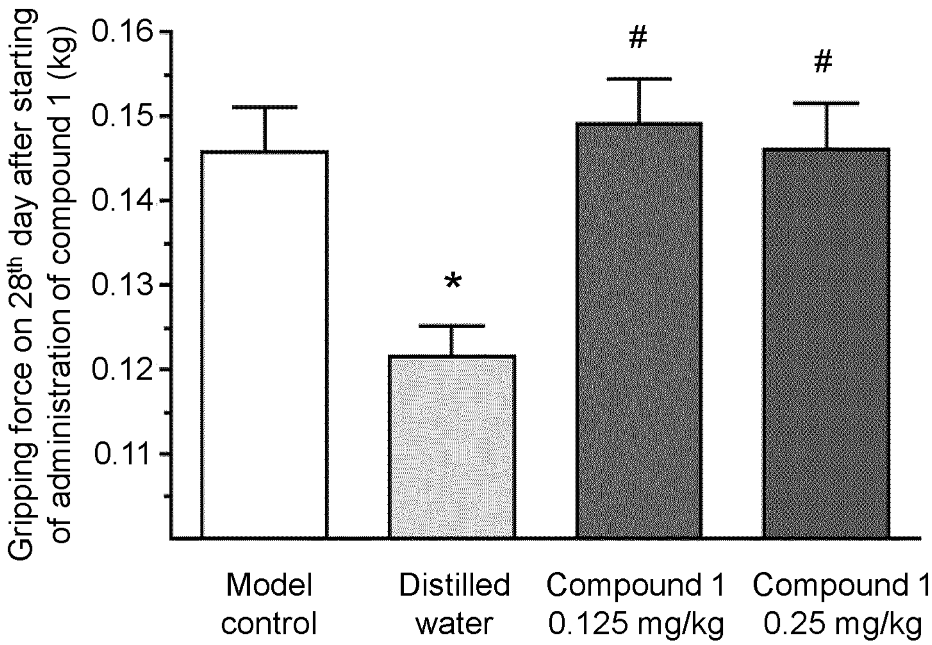
Gripping force on 28th day after starting of administration of compound 1 (kg)
0.16
0.15
0.14
0.13
0.12
0.11
#
#
*
Model control
Distilled water
Compound 1 0.125 mg/kg
Compound 1 0.25 mg/kg

Fig. 3

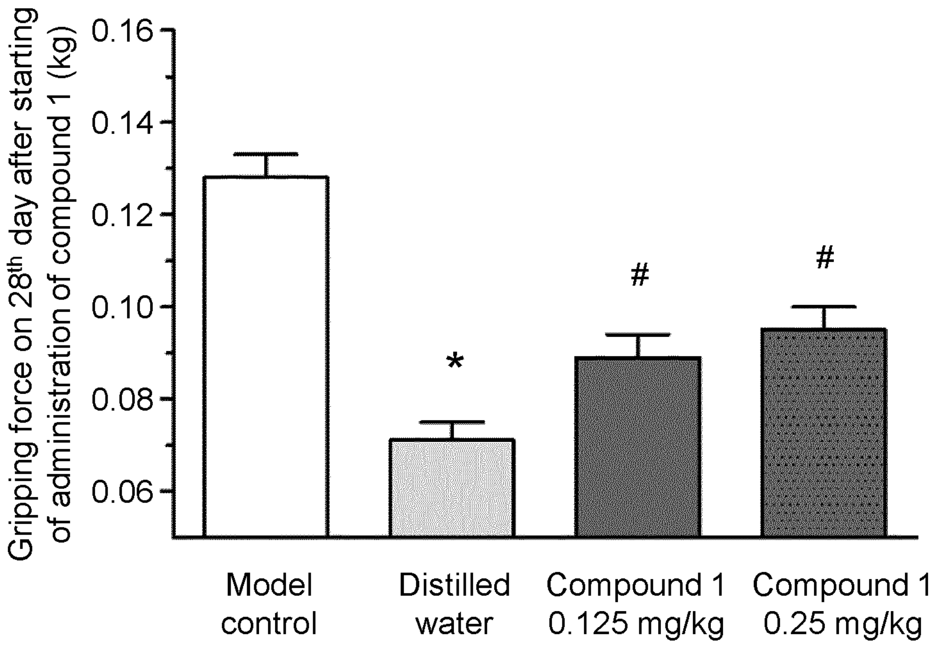
Gripping force on 28th day after starting of administration of compound 1 (kg)
0.16
0.14
0.12
0.10
0.08
0.06
*
#
#
Model control
Distilled water
Compound 1 0.125 mg/kg
Compound 1 0.25 mg/kg

**INTERNATIONAL SEARCH REPORT**

International application No.
PCT/JP2021/024759

A. CLASSIFICATION OF SUBJECT MATTER
A61K 31/485(2006.01)i; A61P 3/00(2006.01)i; A61P 21/00(2006.01)i
FI: A61K31/485; A61P3/00; A61P21/00

According to International Patent Classification (IPC) or to both national classification and IPC

B. FIELDS SEARCHED

Minimum documentation searched (classification system followed by classification symbols)
A61K31/485; A61P3/00; A61P21/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | WO 93/15081 A1 (TORAY INDUSTRIES, INC.) 05 August 1993 (1993-08-05) claims 1, 25, page 1, line 8 from the bottom to page 2, line 10, page 273, pp. 394-398, examples 141-142 | 1-6 |
| Y | YOUNG, C. Jessica et al., "Medical Use of Long-term Extended-release Opioid Analgesics in Commercially Insured Adults in the United States", Pain Medicine, 2019, vol. 21, no. 4, pp. 724-735, abstract, table 2 | 1-6 |
| Y | O'BRIEN, Tony et al., "European Pain Federation position paper on appropriate opioid use in chronic pain management", European Journal of Pain, 2017, vol. 21, no. 1, pp. 3-19, abstract, "10. Managing long-term use of opioids", paragraph [0002], | 1-6 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 August 2021 (17.08.2021) | 24 August 2021 (24.08.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/024759

C (Continuation). DOCUMENTS CONSIDERED TO BE RELEVANT

| Category* | Citation of document, with indication, where appropriate, of the relevant passages | Relevant to claim No. |
|---|---|---|
| A | JP 2017-520622 A (KOREA RESEARCH INSTITUTE OF BIOSCIENCE AND BIOTECHNOLOGY) 27 July 2017 (2017-07-27) claims 1, 10, examples, in particular, examples 1-2, 5 | 1-6 |
| A | WO 2015/166887 A1 (SUNTORY HOLDINGS LIMITED) 05 November 2015 (2015-11-05) claims, examples 1-3, fig. 1-4 | 1-6 |

Form PCT/ISA/210 (continuation of second sheet) (January 2015)

**INTERNATIONAL SEARCH REPORT**
Information on patent family members

International application No.
PCT/JP2021/024759

| Patent Documents referred in the Report | Publication Date | Patent Family | Publication Date |
|---|---|---|---|
| WO 93/15081 A1 | 05 Aug. 1993 | EP 0577847 A1<br>claims 1, 25, page 3, lines 19-33, page 96, pp. 155-158, examples 141-142<br>US 6277859 B1 | |
| JP 2017-520622 A | 27 Jul. 2017 | US 2017/0112817 A1<br>WO 2015/190643 A1<br>EP 3156055 A1 | |
| WO 2015/166887 A1 | 05 Nov. 2015 | US 2017/0042924 A1<br>EP 3138570 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02066475 A
- WO 93015081 A
- WO 98023290 A
- WO 12105475 A
- WO 16152965 A
- JP 2020112486 A
- JP 2021097684 A


### Non-patent literature cited in the description


- **LYNCH et al.** *Pharmacology & Therapeutics,* 2007, vol. 113, 461-487
- **CRUZ-JENTOFT et al.** *Age and Ageing,* 2014, vol. 43, 748-759
- *Chronic Respiratory Disease,* 2014, vol. 11, 247-255
- *The journal of physiology,* 2006, vol. 574, 85-93
- *Journal of Japan Society of Nutrition and Food Science,* 1993, vol. 46, 451-458
- *The American Journal of Clinical Nutrition,* 2013, vol. 97, 7-14
- *Science Advances,* 2019, vol. 5, 7802
- *Lipids,* 2013, vol. 48, 1135-1143
- *Oncology Reports,* 2011, vol. 25, 189-193
- *Journal of Applied Physiology,* 2019, vol. 126, 170-182
- *Disease Models & Mechanisms,* 2017, vol. 10, 787-796
- *Bioscience,* 1979, vol. 29, 168-172
- *Journal of Physical Therapy Science,* 2011, vol. 26, 255-258
- *Annals of Rehabilitation Medicine,* 2017, vol. 41
- *Journal of Cachexia, Sarcopenia and Muscle,* 2018, vol. 9, 975-986
- *Neurotoxicology and Teratolog,* 2003, vol. 25, 543-553
- *The Lancet Oncology,* 2016, vol. 17, 519-531
- *The Lancet Oncology,* 2013, vol. 14, 335-345